# EUROPÄISCHE PATENTANMELDUNG

(11) **EP 2 309 267 A1**
(43) Veröffentlichungstag der Anmeldung: **13.04.2011**
(21) Anmeldenummer: 10009455.6
(22) Anmeldetag: 10.09.2010
(51) Int. Cl.: G01N 33/543, G01N 33/68

(54) **Verfahren zur Herstellung eines Allergietestträgers für die In-vitro-Diagnostik**

(30) Priorität: 09.10.2009 EP 09012801
(71) Anmelder: Merck Patent GmbH, 64293 Darmstadt (DE)
(72) Erfinder: Wahl, Rudiger, 22085 Hamburg (DE); Röseler, Stefani, 50321 Brühl (DE)

(57) **Zusammenfassung**

Die Erfindung betrifft ein Verfahren zur Herstellung eines Allergietestträgers für die In-vitro-Diagnostik, ein mithilfe dieses Verfahrens hergestellten Allergietestträger, insbesondere einen nativen Allergietestträger, die Verwendung des Allergietestträgers zur In-vitro-Diagnostik und ein Verfahren zur In-vitro-Allergiediagnostik.

## Beschreibung

### Gebiet der Erfindung

Die Erfindung betrifft ein Verfahren zur Herstellung eines Allergietestträgers für die In-vitro-Diagnostik, ein mithilfe dieses Verfahrens hergestellten Allergietestträger, insbesondere einen nativen Allergietestträger, die Verwendung des Allergietestträgers zur In-vitro-Diagnostik und ein Verfahren zur In-vitro-Allergiediagnostik.

### Hintergrund der Erfindung

Der Allergologe kennt verschiedene Methoden der In-vivo- und In-vitro-Allergiediagnostik. Der In-vitro-Allergiediagnostik kommt neben der In-vivo-Allergiediagnostik, die beispielsweise Haut-, Prick-, Intracutan- und Provokationstests umfasst, eine immer größere Bedeutung zu, da das Allergenspektrum sich stets vergrößert und eine In-vivo-Testung in vielen Fällen aufgrund von Nebenwirkungen für den Patienten nicht durchführbar ist.

Bei der In-vivo-Allergiediagnostik wie dem Haut-Prick-Test, Intracutantest, der nasalen-, bronchialen, konjunktivalen und oralen Provokation (9, 13) werden Extrakte aus Allergien auslösenden Pflanzen, Nahrungsmitteln, Tierhaaren und dergleichen gewonnen und diese nach einem der genannten Testverfahren dem Patienten appliziert oder die Allergene werden nativ, das heißt beispielsweise eine Allergien auslösende Frucht direkt getestet.

Eine häufig angewandte Methode ist die orale Provokation. Dabei nimmt der Patient unter ärztlicher Aufsicht steigende Konzentrationen von dem betreffenden Nahrungsmittel ein. Wenn eine Allergie vorliegt, wird es dabei zu den typischen Symptomen einer Nahrungsmittelallergie kommen. Die allergischen Symptome bzw. Nebenwirkungen solcher Testverfahren sind mit Risiko behaftet und führen zu einer geringen Akzeptanz beim Patienten.

Ein weiteres In-vivo-Verfahren ist der sogenannte Hauttest oder die kutane Allergiediagnostik. In der klinischen Routine spielt insbesondere die Diagnostik von Nahrungsmitteln eine immer größer werdende Rolle. Während Pollen sehr verlässlich sowohl im Hauttest, als auch serologisch, das heißt in-vitro auf spezifische IgE-Antikörper bei Allergikern hin untersucht werden können, sind Lebensmittel aufgrund der Vielfalt und des reifeabhängigen Allergenpotentials mit kommerziellen Methoden unzulänglich erfassbar. Nahrungsmittel werden deshalb meist nativ in-vivo getestet, da die zur Verfügung stehenden kommerziellen Extrakte wenig verlässliche Ergebnisse liefern. Allergologen bevorzugen somit native Nahrungsmittel zur kutanen Diagnostik. Die Testung erfolgt als "Prick to Prick", d.h. die Pricknadel wird direkt in die Frucht geführt und dann wird der Patient geprickt. Oder die Testung erfolgt als Scratch-Test. Dabei wird das Nahrungsmittel direkt auf ein gescratchtes Hautareal aufgetragen.

Bei Nahrungsmitteln, die der Allergologe nicht kennt, erfolgt eine Kontrolle an einem Nichtallergiker um unspezifische Hautreaktionen, wie beispielsweise durch biogene Amine und Salze zu differenzieren. Nahrungsmittel die biogene Amine enthalten, wie beispielsweise Tomate, Kiwi, Banane können im Hauttest nicht getestet werden, wohl aber zu Allergien führen (11). Sie sind ausschließlich durch die In-vitro-Diagnostik nachweisbar, die jedoch bei Allergologen als unzuverlässig eingestuft wird. Findet der Allergologe in einem Test eine IgE-Sensibilisierung, so ist die klinische Relevanz überprüfbar, findet er keine Sensibilisierung, so ist eine Allergie jedoch nicht auszuschließen.

Die kutane Diagnostik ist somit mit einem Risiko für den Patienten behaftet, nicht für alle Lebensmittel möglich und in der Ergebnisinterpretation nicht immer einfach. Letztendlich muss eine orale Provokation die tatsächliche klinische Relevanz klären, vor der sich viele Patienten und Ärzte scheuen und die auch keine letztendliche Sicherheit gibt.

Das Allergenspektrum erweitert sich ständig. Für zahlreiche neue Allergene stehen zudem vielfach keine kommerziellen Testlösungen zur Verfügung, da der Aufwand für deren Herstellung und für die Erfüllung von regulatorischen Vorgaben nicht im Verhältnis zur Patientenzahl steht, wenn es sich um seltene Allergien handelt. Zu nennen wären hier unter anderem die Berufs- und Hobby-Allergene, wie sie beispielsweise in Hölzern, Rohkaffee, Hygienegebrauchsartikeln wie z.B. Latexhandschuhen und in Fliegenmaden als Angelköder vorkommen, die oft nur kleine Gruppen betreffen. Bei hochgradigen Allergikern kann es zudem bei In-vivo-Testungen mit neuen, noch nicht ausreichend charakterisierten Allergenlösungen zu unzumutbaren Nebenwirkungen kommen, die das Wohlbefinden erheblich beeinträchtigen.

Weiterhin gibt es einige giftige, ätzende bzw. aggressive Allergene (z.B. Isozyanate), die auf der Haut nicht bzw. nur eingeschränkt testbar sind. Darüber hinaus gibt es Bedingungen, bei denen eine Hauttestung schwierig durchzuführen ist, wie bei Säuglingen oder Kleinkindern und bei Patienten mit Hautveränderungen wie Urticaria factitia.

Dies führt sowohl bei Patienten, wie auch bei Ärzten zu Unsicherheit, so dass ein hoher Bedarf an der Bereitstellung von Diagnoseverfahren besteht, die eine sichere Aussage zu bestehenden Allergien eines Patienten zulassen und nicht die Nachteile der In-vivo-Testverfahren wie die schlechte Akzeptanz durch den Patienten, die Nebenwirkungen und die Unsicherheit der Diagnose aufweisen.

Die In-vitro-Diagnostik hat gegenüber den In-vivo-Testverfahren den Vorteil, dass sie für den Patienten völlig ungefährlich ist, da die Testung mit dem Serum des Patienten außerhalb des Organismus durchgeführt wird. Zur Testung eines Allergens sind 50 µl Serum/Plasma erforderlich, das heißt mit nur einem Milliliter Serum kann auf zwanzig verschiedene Allergene getestet werden. So nimmt die In-vitro-Allergie-Diagnostik einen wichtigen Stellenwert im Rahmen der Allergie-Diagnostik ein (2, 4, 5, 8, 17).

Im Rahmen der In-vitro-Allergiediagnostik spielt das IgE (Immunglobulin E) die entscheidende Rolle. IgE wurde 1967, jeweils unabhängig von einander, von Johannson und dem Forscherpaar Ishizaka entdeckt. Auch entwickelten Wide, Bennich und Johannson das erste Testsystem, um spezifisches IgE in Seren von Allergikern zu messen. Dies war das Allergenscheiben-Testsystem (1, 15), das auch heute noch häufig in der In-vitro-Allergiediagnostik eingesetzt wird. Dieses System ermöglicht, eine Vielzahl von Allergenen an den Träger, eine chemisch aktivierte Papierscheibe, zu koppeln (Allergenscheibe). Heute stehen allerdings auch andere Testsysteme zur Verfügung (2), die teilweise auf unterschiedlichen Allergenträgern beruhen, wie ELISA-Systeme, das CAP-System (16) oder Flüssigallergen-Systeme (3).

In-vitro-Allergenscheiben werden überwiegend nach der Methode von Ceska et al. und Wide et al. (1, 15) hergestellt. Dieses Verfahren stellt den heute am häufigsten verwendeten Test dar. Es ist gleichzeitig das erste Testsystem, um spezifisches IgE in Seren von Allergikern zu messen. Diese Technik ist sehr umfangreich, arbeits- und zeit intensiv. Zunächst werden Proteine bzw. Allergene aus einem Extrakt an ein mit Bromcyan aktiviertes Papier kovalent gebunden, dann werden durch Waschung mit einem Ethanolamin enthaltenden Puffer freie Aminogruppen blockiert und schließlich wird mit einem Essigsäure enthaltenden Puffer erneut gewaschen. Details zu den Pufferzusammensetzungen und den Waschungen gemäß der Methode von Ceska et al. (1) sind Beispiel 1 zu entnehmen. Ähnliche Testsysteme sind beispielsweise in der EP 203 238 beschrieben.

Außer an chemisch aktivierte Papierscheiben können Allergene auch an Polystyrolkügelchen, Mikrotiterplattenkavitäten oder Polystyrolröhrchen adsorptiv oder nach Vorbehandlung der Träger auch kovalent gebunden werden. Wenige Tests beinhalten die Allergenkopplung an Nitrocellulose. ELISA-Systeme basieren überwiegend darauf, dass z.B. Allergene nur adsorptiv an eine Polystyroloberfläche gebunden werden. Der Einsatz dieses Systems ist für die native Testung von Allergenen nicht möglich.

In der WO 85/02262 werden Streifen (Sticks) beschrieben, an die verschiedene Allergenen gebunden sind und die zum Screenen von Humanserum mittels klassischem RAST (Radioallergosorbent test) dienen. In der US 4,331,650 wird ein Agglutinationstest zur Bestimmung von Immunglobulinen im Blutserum beschrieben. In DE 1952676 wird eine Streifentestanalyse zur Erfassung von autoallergen menschlichen Pathologien offenbart.

Beim Cap-System werden die Allergene an den CAP, einem in einer kleinen Kapsel befindlichen Polymer, kovalent gebunden werden. Auch hier erfolgt die Kopplung der Allergene an eine BrCN-aktivierten Träger-Sepharose, gefolgt von mehreren Waschschritten. Auch Flüssigallergene werden in Testsystemen eingesetzt (z.B. DPC Siemens). Ebenfalls ist die Kopplung der Allergene an magnetische Partikel möglich.

Das Funktionsprinzip ist bei allen gängigen Systemen dasselbe, das jeweilige Allergen reagiert mit dem spezifischen IgE aus dem Patientenserum, die einzelnen Systeme unterscheiden sich jedoch beispielsweise in der Selektivität der Kopplung der Allergene und der Menge der auf der Oberfläche des Trägers gebundenen Allergene.

Bei allen bekannten In-vitro-Testverfahren geht man von Extrakten aus, das heißt, die Testung erfolgt nicht-nativ. Die extrahierten Allergene werden überwiegend kovalent oder adsorbtiv an entsprechende Allergenträger gekoppelt. Schon bei der Allergenextraktherstellung, die bei der Herstellung der Allergietestträger vorgeschaltet ist, können eventuell Proteine bzw. Allergene denaturieren oder es müssen dem Extrakt Substanzen zugesetzt werden, die diesen häufig auftretenden enzymatischen Abbau der Proteine bzw. Allergene verhindern (7). Das trifft insbesondere häufig auf Nahrungsmittelextrakte zu, da diese häufig thermolabile Proteine enthalten, die durch die normale Extraktherstellung zerstört werden und so ihre allergene Aktivität verlieren (7, 14). So müssen zur optimalen Apfelextraktherstellung Tieftemperaturextrakte eingesetzt werden (10, 11), deren Herstellung sehr kompliziert ist.

Pastor et al. (7) zeigen, dass die Hauptallergene der Wassermelone überwiegend Enzyme sind. Aus diesem Grunde müssen bei der Extraktherstellung der Wassermelone Antioxidantien zugesetzt werden. Auch bei eigenen, hier gezeigten Untersuchungen war das Allergen beispielsweise mit dem Serum eines Allergikers, der eine Anaphylaxie, das heißt eine lebensbedrohliche Schwellung der Mundschleimhäute und des Kehlkopfes nach Honigmelonenverzehr zeigte, serologisch (CAP-RAST In-vitro-Diagnostik) nicht nachweisbar. Im Hauttest zeigte derselbe Patient eine eindeutige Sensibilisierung. So ist anzunehmen, dass es bei der Extraktherstellung zur Denaturierung der Honigmelonenallergene und zum Verlust allergener Aktivität kommt. Folglich wird die Qualität dieser Allergietestträger sehr stark von der Qualität der Extrakte beeinflusst, die zur Kopplung an das Trägermaterial eingesetzt werden und die Extrakte sollten möglichst auf molekularer Basis charakterisiert werden (6).

Mit den bislang bekannten In-vitro-Testverfahren war es also nicht möglich spezifische IgE-Antikörper direkt mit dem potentiell Allergene enthaltenden Stoff, das heißt nativ, zu testen, sondern immer nur über den Umweg der Extraktherstellung (16), mit den oben genannten Nachteilen und Problemen. Da zudem kommerzielle Anbieter von Allergietestsystemen verständlicherweise nur solche Testungen zur Verfügung stellen, deren Allergene bekannt und in entsprechender Häufung kommerziell vielversprechend sind, steht bislang nur eine begrenzte Menge von In-vitro-Testsystemen zur Verfügung. Hier muss überdies das weite Feld der Kreuzreaktionen mit berücksichtigt werden, so sind Birkenpollenallergiker auch häufig Apfelallergiker (6), die teilweise nicht mit dem entsprechenden Testextrakt in-vitro getestet werden können.

Darüber hinaus weisen alle bekannten In-vitro-Testsysteme den Nachteil der aufwendigen und langwierigen Durchführung auf. Insbesondere die Kopplung des Allergens an den Allergietestträger und die spätere Auswertung kann meist nicht von jedermann auf einfache Weise ausgeführt werden. Zudem können solche Testssysteme häufig nicht sowohl im human-medizinischen Bereich, als auch in der Veterinärmedizin zur Diagnose eingesetzt und gleichermaßen mit Serum und Vollblut durchgeführt werden, ohne dass hierbei das Ergebnis verfälscht wird.

Zusammenfassend lässt sich festhalten, dass alle bisher bekannten In-vivo-Testsysteme wesentliche Nachteile, wie eine schlechte Akzeptanz durch den Patienten, Nebenwirkungen und die Unsicherheit der Diagnose aufweisen.

Die bislang bekannten In-vitro-Testsysteme weisen ebenfalls eine hohe Unsicherheit der Diagnose durch die Extraktherstellung auf, es ist nur eine sehr begrenzte Zahl von verschiedenen Allergenen käuflich erhältlich, sie sind meist nur für einen Fachmann durchführbar und ein Test direkt mit dem potentiell Allergene enthaltenden Stoff, das heißt ein natives In-vitro-Testsystem ist bislang nicht verfügbar. Insbesondere ist bisher kein In-vitro System verfügbar, womit in der Arztpraxis gezielt beispielsweise auf das Nahrungsmittel hin getestet werden kann, auf das der Patient allergische Reaktionen zeigte. Einen hohen Bedarf an einem solchen einfach durchzuführenden und schnellen Verfahren zeigt auch eine Veröffentlichung im Laborpraxis Newsletter 2009 (6).

Die der vorliegenden Erfindung zugrund liegende Aufgabe bestand nun in der Bereitstellung eines Allergietestträgers der insbesondere bei der nativen In-vitro-Allergiediagnostik eingesetzt werden kann.

### Beschreibung der Erfindung

Überraschend wurde nun ein Verfahren zur Herstellung eines Allergietestträgers für die In-vitro-Diagnostik gefunden, der die obengenannten Nachteile nicht aufweist.

Gegenstand der Erfindung ist ein Verfahren zur Herstellung eines Allergietestträgers für die In-vitro-Diagnostik, **dadurch gekennzeichnet, dass**
a.) ein aktivierter Träger mit einem Allergene enthaltenden Stoff in Kontakt gebracht wird,
b.) die Allergene an den aktivierten Träger koppeln,
c.) nach Kopplung der Allergene an den aktivierten Träger keine Blockierung erfolgt
d.) und der erhaltene Allergietestträger direkt zur Messung der Bindung von spezifischen IgE-Antikörpern aus dem Serum oder Vollblut eines Patienten verwendet wird,
e.) oder der erhaltene Allergietestträger bis zur Messung der Bindung von spezifischen IgE-Antikörpern aus dem Serum oder Vollblut eines Patienten aufbewahrt wird.

Allergene enthaltende Stoffe gemäß der vorliegenden Erfindung sind beispielweise:
a.) Nahrungsmittel wie beispielsweise Früchte, Gemüse, Salat, Fleisch von Haus- und Wildtieren, Vögeln, Meerestieren und dergleichen, Milch, Mehle, Wein, Bier, Kaffee, Tee, Eier, Soja und dergleichen,
b.) Körperpflegemittel wie beispielsweise Cremes, Shampoos, Seifen, Lippenstifte, Zahnpasta, Haarpflegemittel, Sonnenschutzmittel und dergleichen
c.) Teile von Pflanzen wie beispielsweise Pollen, Früchte, Nüsse, Blüten, Hölzer, Blätter, Wurzeln und dergleichen sowie Teile von Pilzen, wie beispielsweise Schimmelpilzen und dergleichen,
d.) Tierhaare, beispielsweise von Hund, Katze, Pferd, Rind und weiteren Haus- und Wildtieren und dergleichen,
e.) Federn von Vögeln,
f.) Körperflüssigkeiten, Epithelien und Ausscheidungen von Tieren, beispielsweise von Hund, Katze, Pferd und Rind und weiteren Haus- und Wildtieren, Vögeln, Meerestieren, Insekten und dergleichen, wie beispielsweise Speichel, Kot, wie beispielsweise von Milben, Urin, Schweiß, Blut, Katzenepithelien und dergleichen,
g.) Schaben,
h.) Insektengifte wie beispielsweise Wespengifte, Bienengifte und dergleichen,
i.) Hygienegebrauchsartikel wie beispielsweise Latexhandschuhe und dergleichen,
j.) Stoffe, die Haptene enthalten und eine allergische Reaktion auslösen können, wie beispielsweise Medikamente, beispielsweise die Antibiotika Penicillin und Amoxicillin, Kontrastmittel und dergleichen,
k.) Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen.

Erfindungsgemäße Allergene enthaltende Stoffe sind auch Extrakte aus dem den obengenannten Stoffen.

Grundsätzlich kann jedes Protein ein Allergen bilden. Somit umfassen erfindungsgemäße Allergene enthaltende Stoffe auch alle Stoffe die Proteine enthalten sowie alle Stoffe, die Allergene oder Haptene enthalten, die nicht explizit oder implizit in der obenstehenden Aufstellung offenbart sind.

Erfindungsgemäß sind demnach auch Haptene, die allergische Reaktionen bei Allergikern auslösen können. Dies sind chemische Stoffe, die meist nicht direkt sondern beispielsweise über Humanserum-Albumin oder Polylysin an den aktivierten Träger gekoppelt werden. Haptene finden sich beispielsweise in vielen Medikamenten wie beispielsweise in den Antibiotika Penicillin und Amoxicillin, in Kontrastmitteln und dergleichen.

Beim erfindungsgemäßen Allergene enthaltenden Stoff handelt es sich um einen der genannten Stoffe, der ein Allergen oder Hapten oder mehrere verschiedene Allergene oder Haptene enthalten kann.

Ein bevorzugter Gegenstand der Erfindung ist somit das erfindungsgemäße Verfahren, **dadurch gekennzeichnet, dass** es sich bei dem Allergene enthaltenden Stoff um Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten, oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen oder um Extrakte aus Nahrungsmitteln, Körperpflegemittein, Teilen von Pflanzen und Pilzen, Tierhaaren, Federn, Körperflüssigkeiten, Epithelien und Ausscheidungen von Tieren, Schaben, Insektengiften, Hygienegebrauchsartikein, Stoffen, die Haptene enthalten, oder Stoffen, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

Ein weiter bevorzugter Gegenstand der Erfindung ist das erfindungsgemäße Verfahren, **dadurch gekennzeichnet, dass** es sich bei dem Allergene enthaltenden Stoff um Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten, oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt. Ein weiterer bevorzugter Gegenstand der Erfindung ist das erfindungsgemäße Verfahren, **dadurch gekennzeichnet, dass** auch gekochte Proteine, wie beispielsweise vom Hühnerei oder der Kartoffel nativ an den Allergietestträger gekoppelt werden können.

Ein besonders bevorzugter Gegenstand der Erfindung ist demnach das erfindungsgemäße Verfahren, **dadurch gekennzeichnet, dass** es sich bei dem Allergietestträger um einen nativen Allergietestträger handelt.

Im Sinne der Erfindung bedeutet "nativ" bzw. "nativer Allergietestträger", dass man den aktivierten Träger direkt mit dem Allergene enthaltenden Stoff in Kontakt bringt und direkt im Anschluss die Messung der Bindung von spezifischen IgE-Antikörpern aus dem Serum oder Vollblut eines Patienten durchführt oder den Allergietestträger bis zur Messung der Bindung von spezifischen IgE-Antikörpern aus dem Serum oder Vollblut eines Patienten aufbewahrt. Im Gegensatz dazu, bedeutet "nicht nativ", dass man Allergietestträger mithilfe von Extrakten und anderen Aufbereitungen, Aufreinigungen oder Isolierungen aus Allergene enthaltenden Stoffen herstellt.

Ein weiterer Gegenstand der Erfindung ist ein Verfahren zur Herstellung eines Proteintestträgers für die In-vitro-Diagnostik, **dadurch gekennzeichnet, dass**
a.) ein aktivierter Träger mit einem Proteine enthaltenden Stoff in Kontakt gebracht wird,
b.) die Proteine an den aktivierten Träger koppeln,
c.) nach Kopplung der Proteine an den aktivierten Träger keine Blockierung erfolgt
d.) und der erhaltene Proteintestträger direkt zur Messung oder zum Nachweis der gebundenen Proteine verwendet wird,
e.) oder der erhaltene Allergietestträger bis zur Messung oder zum Nachweis der gebundenen Proteine aufbewahrt wird.

Demgemäß ist die Verwendung der vorliegenden Erfindung nicht nur auf das Gebiet der Allergologie beschränkt, sondern findet auch Anwendung beim Nachweis und Messung von Proteinen in weitere denkbaren technischen Gebieten, wie beispielweise in der Labordiagnostik wissenschaftlicher Einrichtungen, Hygienediagnostik, bei der Herstellung von Stoffen, die Proteinen enthalten, beim Nachweis von Proteinen im Nahrungsmittelbereich, bei der Gewässer- und Umweltanalyse und im Veterinärbereich und weiteren technischen Gebieten, bei denen der Nachweis und die Messung von Proteinen eine wesentliche Rolle spielen. Der erfindungsgemäße Proteintestträger dient hierbei als Proteinfänger, der die Proteine beispielsweise aus Flüssigkeiten oder von Oberflächen bindet und damit für Nachweise und Messungen verfügbar macht.

Zur Herstellung der erfindungsgemäßen Allergietestträger werden die Allergene kovalent und damit sehr stabil an chemisch aktivierte Träger gekoppelt. Eine kovalente Kopplung ist deshalb möglich, weil eine chemische Aktivierung der Träger (Durchmesser 6 mm) beispielsweise mittels Bromcyan (BrCN) erfolgt. Dadurch wird die Bindung der freien NH₂-Gruppen der Allergene mit den CN-Gruppen der Festphase ermöglicht. Nicht nur die häufigsten und relevantesten Allergene wie beispielsweise Milben, Katzenepithelien, Schimmelpilze lassen sich an das Trägermaterial koppeln, sondern auch einige Haptene, beispielsweise aus Medikamenten. Hier erfolgt die Kopplung nicht direkt, sondern beispielsweise über Humanserum-Albumin oder Polylysin. Das Trägermaterial ist auch zur Kopplung von Allergenmischungen geeignet. Solche Allergietestträger werden Multi-, Misch- oder Sammelallergentestträger genannt.

Zur Kopplung der Allergene an den aktivierten Träger reicht beispielsweise schon eine Kontaktdauer von einer Sekunde, vorzugweise wird der aktivierte Träger 5 Sekunden bis über Nacht, bevorzugt 30 Sekunden bis 15 Minuten, besonders bevorzugt 1 bis 5 Minuten mit dem Allergene enthaltenden Stoff in Kontakt gebracht (siehe Beispiel 6).

In den in den Beispielen aufgeführten Untersuchungen wurden BrCN-aktivierte Papierscheiben eingesetzt. Diese aktivierten Träger werden gegebenenfalls leicht angefeuchtet und beispielweise direkt auf das Fruchtfleisch oder auf das Mehl gelegt, werden mit einer Pinzette gehalten und über das Fell eines Tieres gestrichen, werden in die Flüssigkeit, beispielweise Hundespeichel oder Nagerurin, gehalten oder werden über Schimmelpilze oder Polster (Milbenkot) gestrichen oder aufgelegt. Dabei werden die Allergene an den aktivierten Träger gebunden.

Die Denaturierung der Allergene, wie beispielsweise bei der Extraktherstellung für herkömmliche Allergietestträger, ist bei der Herstellung nativer Allergietestträger nicht notwendig. Dadurch sind die erfindungsgemäßen nativen Allergietestträger den käuflich erhältlichen Allergietestträgern deutlich überlegen bzw. viele Allergene bzw. Allergien lassen sich erst mit dem erfindungsgemäßen System nachweisen.

Direkt nach Kopplung der Allergene an den aktivierten Träger kann der erfindungsgemäße Allergietestträger direkt zur Messung der Bindung von spezifischen IgE-Antikörpern aus dem Serum oder Vollblut eines Patienten verwendet werden.

Die Bedingungen für die Kopplung des Allergene enthaltenden Stoffs an den aktivierten Träger sind reproduzierbar (siehe Beispiel 8) und einfach in allergologischen Fachlaboren durchzuführen. So ergaben die hierzu durchgeführten Untersuchungen am Beispiel von Zwiebel, Kiwi, Apfel und Honigmelone, dass die erfindungsgemäßen und insbesondere die nativen Allergietestträger sinnvoll zur spezifischen IgE-Messung eingesetzt werden können und reproduzierbare Ergebnisse liefern. Auch wies die Messung der Klassen über drei unabhängige Messungen eine hohe Reproduzierbarkeit auf (siehe Beispiel 7).

Bei erfindungsgemäßen Materialien, die sich als Träger eignen, handelt es sich beispielsweise um Papier, Nitrocellulose, PVDF-Membran, Sepharose, Cellulose, Polystyrol und dergleichen.

Diese Träger können in Scheibenform, als Plättchen, Kugeln oder als Streifen und dergleichen vorliegen oder zu solchen geschnitten oder geformt werden.

Erfindungsgemäß ist demnach ein Verfahren, **dadurch gekennzeichnet, dass** der Träger aus Papier, Nitrocellulose, einer PVDF-Membran, Sepharose, Cellulose, Polystyrol und dergleichen besteht.

Die Aktivierung der Träger erfolgt beispielsweise durch Lösungen, die Bromcyan, Epoxy und dergleichen enthalten oder durch Diazotierung und dergleichen. Zur Aktivierung wird der erfindungsgemäße Träger, wie beispielsweise Papier, beispielweise mit einer Bromcyan enthaltenden Lösung inkubiert. Dabei kommt es zur Ausbildung von CN-Gruppen über die Reaktion des Bromcyans mit den OH-Gruppen des Papiers.

Somit ist ein weiterer Gegenstand der Erfindung ein erfindungsgemäßes Verfahren, **dadurch gekennzeichnet, dass** der Träger mithilfe von Bromcyan oder Expoxy enthaltenden Lösungen oder durch Diazotierung aktiviert wird oder ist.

Überraschenderweise ist im Falle des erfindungsgemäßen Verfahrens im Gegensatz zu den bisher bekannten Herstellungsverfahren nach Kopplung der Allergene an den aktivierten Träger keine Blockierung erforderlich (siehe Beispiel 1). Dieser und weitere Verfahrensschritte sind bei der Herstellung von herkömmlichen, kommerziell erhältlichen Allergietestträgern zur In-vitro-Diagnostik kompliziert, zeitlich aufwendig und teuer. Beispielweise sollen bei der Methode nach Ceska et al. (1) in einem ersten Schritt mit einem Ethanolamin enthaltenden Puffer möglicherweise noch freie Bindungsstellen blockiert werden. Bei Weglassen der Blockierung würde der Fachmann erwarten, dass Proteine wie insbesondere IgE-Antikörper aus dem Serum des Patienten binden und so ein falsch positives Ergebnis liefern.

Weitere Puffer und Lösungen, die zur Blockierung geeignet sind, sind neben Puffern und Lösungen, die Ethanolamin enthalten, Puffer und Lösungen, die beispielsweise andere wasserlösliche primäre Amine, wie beispielsweise Aminosäuren, oder Gelatine, Molkepulver oder dergleichen enthalten.

Ein weiterer Gegenstand der Erfindung sind somit erfindungsgemäße Verfahren, **dadurch gekennzeichnet, dass** nach Kopplung der Allergene an den aktivierten Träger keine Blockierung mit Puffern oder Lösungen, die primäre Amine, Gelatine, Molkepulver oder dergleichen enthalten, erfolgt.

Gemäß den hier vorgestellten Untersuchungsergebnissen ist es beim erfindungsgemäßen Verfahren nach Kopplung der Allergene nicht notwendig, den erfindungsgemäßen Allergietestträger mit Ethanolamin zu blockieren. Allergietestträger, die mit- und ohne Ethanolaminblockierung hergestellt wurden, ergaben vergleichbare Ergebnisse (siehe Beispiel 1). Überraschenderweise werden offenbar bei dem direkten Kontakt mit dem Allergene enthaltenden Stoff schon nahezu alle Bindungsstellen besetzt oder blockiert (siehe Beispiel 9). So kam es in den Untersuchungen gemäß Beispiel 10 auch zu keiner unspezifischen Reaktion und selbst bei der Einstellung des Myeloma IgE-Serums auf 1000 IU/ml wurde eine Klasse 0 gemessen, obwohl bei der Herstellung der erfindungsgemäßen nativen Allergentestträger diese nach der Herstellung nicht mit Ethanolamin blockiert wurden und ein Fachmann eine unspezifische Reaktion erwartet hätte.

In der Methode nach Ceska et al. (1) wird in einem weiteren Schritt mit einem Essigsäure enthaltenden Puffer gewaschen. In diesem Waschschritt werden Reste des Ethanolamins herausgewaschen. Überraschenderweise ergaben jedoch Allergietestträger, die mit- und ohne Waschschritt mit Essigsäure enthaltendem Puffer hergestellt wurden, vergleichbare Ergebnisse (siehe Beispiel 1). Weitere Puffer und Lösungen, die für diesen Waschschritt im Sinne der Methode nach Ceska et al. geeignet wären, sind andere saure Puffer und Lösungen, wie beispielsweise Zitronensäure enthaltende Puffer und dergleichen.

Waschschritte im Sinne der vorliegenden Erfindung sind Waschungen des aktivierten Trägers nach Kopplung der Allergene bzw. des erfindungsgemäßen Allergietestträgers, die dazu dienen, dem Allergietestträger Stoffe zu entziehen oder aufgelagerte Stoffe, beispielsweise das zur Blockierung verwendete Ethanolamin, zu entfernen. Solche Waschschritte sind im erfindungsgemäßen Verfahren nicht notwendig.

Die Lagerung der erfindungsgemäßen Allergietestträger in Aufbewahrungspuffer ist kein Waschschritt im Sinne der vorliegenden Erfindung. Weiterhin sind Handlungen, die zur Messung spezifischer IgE-Antikörper aus dem Serum oder Vollblut eines Patienten und Vorbreitungshandlungen zur Ausführung dieser Messung nicht als Waschschritte im Sinne der Erfindung zu verstehen.

Ein weiterer Gegenstand der Erfindung sind somit erfindungsgemäße Verfahren, **dadurch gekennzeichnet, dass** nach Kopplung der Allergene an den aktivierten Träger keine Waschschritte erfolgen.

Ein weiterer bevorzugter Gegenstand der Erfindung sind somit auch erfindungsgemäße Verfahren, **dadurch gekennzeichnet, dass** nach Kopplung der Allergene an den aktivierten Träger keine Blockierung und keine Waschschritte erfolgen.

Ein weiterer Gegenstand der Erfindung sind erfindungsgemäße Verfahren, **dadurch gekennzeichnet, dass** nach Kopplung der Allergene an den aktivierten Träger keine Blockierung mit Puffern oder Lösungen, die primäre Amine, Gelatine, Molkepulver oder dergleichen enthalten und keine Waschschritte mit sauren Puffern oder Lösungen erfolgen.

Die prozessierten Allergietestträger, insbesondere die erfindungsgemäßen nativen Allergietestträger können somit ohne vorherige Waschung direkt im Test eingesetzt werden. Die Proteine beispielsweise aus Fruchtfleisch reagieren offenbar sofort bei Kontakt mit dem BrCN-aktivierten Träger. Es liegt eine kovalente Bindung vor, das heißt, die NH₂-Gruppe des Proteins bzw. Allergens reagiert mit der CN-Gruppe zu einem stabilen Carbodiimid-Komplex.

Das erfindungsgemäße Herstellungsverfahren ist reproduzierbar, deutlich weniger aufwendig, schneller und preisgünstiger und die Kopplung der Allergene kann insbesondere auch von einem Arzt selbst vor Ort durchgeführt werden. Es ist somit möglich, Allergene aus dem Allergene enthaltenden Stoff direkt, das heißt nativ zu binden und eine Allergie des Patienten direkt, beispielsweise an einer mitgebrachten Frucht zu messen. Zuvor musste der Arzt auf kommerziell erhältliche Allergietestträger zurückgreifen, mit den oben genannten Problemen und Nachteilen der Extraktherstellung und mit dem Problem, dass für viele Allergene überhaupt keine Testsysteme erhältlich waren. Mit dem erfindungsgemäßen Verfahren zur In-vitro-Allergiediagnostik ergibt sich auch für Patienten, die an seltenen Allergien leiden oder an solchen Allergien leiden, zu denen noch kein Testsystem erhältlich ist, ein deutlich erhöhtes Maß an Sicherheit. Beispielsweise kann der Arzt jetzt in den Anaphylaxiepass des Patienten eine Allergie eintragen, mit dem auch Kollegen, die diese Erkrankung oder Allergie nicht kennen, eine gesicherte Diagnostik vorgelegt werden kann, sollte es einmal beispielsweise durch in Lebensmitteln versteckte Allergene beim Patienten zu einer Reaktion kommen. Der Allergiker selbst kann beispielsweise im Restaurant oder im Lebensmittelgeschäft erfragen, ob in einem Lebensmittel versteckte Mengen des Allergens vorhanden sind, da er sicher den Auslöser allergischer Reaktionen kennt, bzw. er kann außerdem ein Notfallset mitführen, um sich selbst Erste-Hilfe zu leisten.

Durch Einsatz der erfindungsgemäßen Allergietestträger, insbesondere der erfindungsgemäßen nativen Allergietestträger kann der Arzt direkt vor Ort auf das Allergen testen, auf das der Patient allergisch reagiert. Der Patient kann also genau das Nahrungsmittel zum Arzt mitbringen, auf das er allergische Reaktionen zeigte und der Arzt kann die Testung sofort durchführen. Zur Herstellung der erfindungsgemäßen Allergietestträger benötigt der Arzt nur den aktivierten Träger, der zur Kopplung des Allergens mit dem Allergene enthaltenden Stoff in Kontakt gebracht wird und ein entsprechendes Messsystem mit dem er spezifische IgE-Antikörper im Vollblut oder Serum des Patienten nachweisen und die Allergieklasse bestimmen kann. Als Kontrolle sollte immer eine Parallelmessung mit einem Nichtallergikerserum erfolgen, um eventuelle falsch positive Reaktionen mit dem Allergietestträger auszuschließen. Mit den erfindungsgemäßen nativen Allergietestträgern eröffnet sich für die Diagnostik eine weitaus größere Spannbreite, als es mit der herkömmlichen Verwendung von handelsüblichen Fertigprodukten möglich ist.

Möchte oder kann der Arzt die Messung spezifischer IgE-Antikörper nicht sofort durchführen, so kann er den Allergietestträger bis zur abschließenden Messung bevorzugt gekühlt lagern, entweder trocken oder in Aufbewahrungspuffer.

Vor Kopplung der Allergene angefeuchtete aktivierte Träger bzw. aktivierte Träger, die in Flüssigkeiten gehalten wurden oder mit eher feuchten Allergene enthaltenden Stoffen in Kontakt gebracht wurden, werden vorzugweise nach der Kopplung der Allergene trocken, beispielsweise in einem Röhrchen gelagert.

Vor Kopplung der Allergene nicht angefeuchtete aktivierte Träger bzw. aktivierte Träger, die mit eher trockenen Allergene enthaltenden Stoffen in Kontakt gebracht wurden, werden bevorzugt in einem Aufbewahrungspuffer gelagert. Erfindungsgemäße Aufbewahrungspuffer sind Puffer wie beispielsweise Kaliumphosphatpuffer und weitere physiologische Puffer wie beispielsweise physiologische Kochsalzlösung und dergleichen. Erfindungsgemäße Aufbewahrungspuffer weisen einen pH-Wert von 3 bis 9, bevorzugt von 4 bis 8, besonders bevorzugt von 5 bis 8 auf.

Jedoch ist in den meisten Fällen wahlweise sowohl eine trockene Lagerung als auch eine Lagerung in Aufbewahrungspuffer möglich.

Erfindungsgemäße Allergietestträger werden bevorzugt bis zur Messung der Bindung von spezifischen IgE-Antikörpern aus dem Serum oder Vollblut eines Patienten trocken oder in erfindungsgemäßen Aufbewahrungspuffern gekühlt, das heißt bei Temperaturen von 0,5°C bis 20°C, bevorzugt bei 1°C bis 15°C, besonders bevorzugt bei 2°C bis 8°C aufbewahrt.

Die erfindungsgemäßen Allergietestträger können unter den vorgenannten Bedingungen wenigstens bis zu einem Jahr, bevorzugt bis zu sechs Monate, besonders bevorzugt bis zu einem Monat gelagert werden (siehe Beispiel 11).

Ein bevorzugter Gegenstand der Erfindung ist somit ein erfindungsgemäßes Verfahren, **dadurch gekennzeichnet, dass** der Allergietestträger bis zur Messung der Bindung von spezifischen IgE-Antikörpern aus dem Serum oder Vollblut eines Patienten trocken oder in Aufbewahrungspuffer gekühlt aufbewahrt wird.

Ein weiterer Gegenstand der Erfindung ist ein Allergietestträger für die In-vitro-Diagnostik, dadurch erhältlich, dass
a.) ein aktivierter Träger mit einem Allergene enthaltenden Stoff in Kontakt gebracht wird,
b.) die Allergene an den aktivierten Träger koppeln,
c.) nach Kopplung der Allergene an den aktivierten Träger keine Blockierung erfolgt
d.) und der erhaltene Allergietestträger direkt zur Messung der Bindung von spezifischen IgE-Antikörpern aus dem Serum oder Vollblut eines Patienten verwendet wird
e.) oder der erhaltene Allergietestträger bis zur Messung der Bindung von spezifischen IgE-Antikörpern aus dem Serum oder Vollblut eines Patienten aufbewahrt wird.

Ein weiterer Gegenstand der Erfindung sind Allergietestträger für die In-vitro-Diagnostik, dadurch hergestellt, dass
a.) ein aktivierter Träger mit einem Allergene enthaltenden Stoff in Kontakt gebracht wird,
b.) die Allergene an den aktivierten Träger koppelt,
c.) nach Kopplung der Allergene an den aktivierten Träger keine Blockierung erfolgt
d.) und der erhaltene Allergietestträger direkt zur Messung der Bindung von spezifischen IgE-Antikörpern aus dem Serum oder Vollblut eines Patienten verwendet wird
e.) oder der erhaltene Allergietestträger bis zur Messung der Bindung von spezifischen IgE-Antikörpern aus dem Serum oder Vollblut eines Patienten aufbewahrt wird.

Ein Allergietestträger im Sinne der vorliegenden Erfindung ist also ein erfindungsgemäßer, aktivierter Träger an den Allergene gekoppelt sind, mit dem Unterschied zu herkömmlichen Allergietestträgern, dass nach Kopplung der Allergene keine Blockierung und/oder keine weiteren Waschschritte erfolgen.

Ein bevorzugter Gegenstand der Erfindung ist somit der erfindungsgemäße Allergietestträger für die In-vitro-Diagnostik, **dadurch gekennzeichnet, dass** es sich bei dem Allergene enthaltenden Stoff um Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel oder Stoffe, die Haptene enthalten und um Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen oder Extrakte aus Nahrungsmitteln, Körperpflegemitteln, Teilen von Pflanzen und Pilzen, Tierhaaren, Federn, Körperflüssigkeiten, Epithelien und Ausscheidungen von Tieren, Schaben, Insektengiften, Hygienegebrauchsartikeln, Stoffen, die Haptene enthalten oder Stoffen, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

Ein weiter bevorzugter Gegenstand der Erfindung ist der erfindungsgemäße Allergietestträger für die In-vitro-Diagnostik, **dadurch gekennzeichnet, dass** es sich bei dem Allergene enthaltenden Stoff um Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel oder Stoffe, die Haptene enthalten und um Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

Ein besonders bevorzugter Gegenstand der Erfindung ist der erfindungsgemäße Allergietestträger für die In-vitro-Diagnostik, **dadurch gekennzeichnet, dass** es sich bei dem Allergietestträger um einen nativen Allergietestträger handelt.

Ein weiterer Geggenstand der Erfindung ist ein Proteintestträger für die In-vitro-Diagnostik, dadurch erhältlich, dass
a.) ein aktivierter Träger mit einem Proteine enthaltenden Stoff in Kontakt gebracht wird,
b.) die Proteine an den aktivierten Träger koppeln,
c.) nach Kopplung der Proteine an den aktivierten Träger keine Blockierung erfolgt
d.) und der erhaltene Proteintestträger direkt eine Messung oder ein Nachweis der gebundenen Proteine erfolgt,
e.) oder der erhaltene Proteintestträger bis zur Messung oder zum Nachweis der gebundenen Proteine aufbewahrt wird.

Darüber hinaus ist die Verwendung eines erfindungsgemäßen Allergietestträgers zur In-vitro-Diagnostik von Allergien ein weiterer Gegenstand der Erfindung.

Ein bevorzugter Gegenstand der Erfindung ist auch die Verwendung eines erfindungsgemäßen Allergietestträgers zur In-vitro-Diagnostik von Allergien auf Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen.

Ein besonders bevorzugter Gegenstand der Erfindung ist auch die Verwendung eines erfindungsgemäßen Allergietestträgers zur In-vitro-Diagnostik von Allergien, dass es sich bei dem erfindungsgemäßen Allergietestträger um einen nativen Allergietestträger handelt.

Ein weiterer Gegenstand der Erfindung ist ein Verfahren zur In-vitro-Diagnostik von Allergien, **dadurch gekennzeichnet, dass**
a.) ein aktivierter Träger mit einem Allergene enthaltenden Stoff in Kontakt gebracht wird,
b.) die Allergene an den aktivierten Träger koppeln,
c.) nach Kopplung der Allergene an den aktivierten Träger keine Blockierung erfolgt
d.) und mit dem erhaltenen Allergietestträger direkt eine Messung der Bindung von spezifischen IgE-Antikörpern aus dem Serum oder Vollblut eines Patienten erfolgt
e.) oder der erhaltene Allergietestträger aufbewahrt wird und anschließend eine Messung der Bindung von spezifischen IgE-Antikörpern aus dem Serum oder Vollblut eines Patienten erfolgt.

Ein bevorzugter Gegenstand der Erfindung ist ein erfindungsgemäßes Verfahren zur In-vitro-Diagnostik von Allergien, **dadurch gekennzeichnet dass** es sich bei den Allergien um Allergien auf Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

Ein besonders bevorzugter Gegenstand der Erfindung ist ein erfindungsgemäßes Verfahren zur In-vitro-Diagnostik von Allergien, **dadurch gekennzeichnet, dass** es sich bei dem Allergietestträger um einen nativen Allergietestträger handelt.

Das erfindungsgemäße Verfahren zur In-vitro-Diagnostik von Allergien ermöglicht es insbesondere Nahrungsmittel ohne Aufbereitung und somit ohne Verlust von Allergenen zu diagnostizieren. Darüber hinaus ist nun jedes Nahrungsmittel mittels In-vitro-Diagnostik erfassbar und nicht nur diejenigen, zu denen von den bisher auf dem Markt befindlichen Anbietern Allergietestträger zur Verfügung gestellt werden. In Deutschland ist, wie in anderen europäischen Ländern, mit einer weiteren deutlichen Zunahme von Nahrungsmittelallergien zu rechnen. Bereits jetzt gibt es regionale Schwankungen von 20% bis 70% bei Birkenpollenallergikern, die eine klinisch relevante Nuss- bzw. Kernobstsensibilisierung (orales Allergiesyndrom) zeigen, so dass eine verlässliche In-vitro-Diagnostik zukünftig eine noch größere Rolle spielen wird. Das erfindungsgemäße Verfahren, erfüllt neben der verlässlicheren und breitflächigen Diagnostik auch die Anforderung nach einem einfach durchzuführenden und relativ schnellen Allergietest. Die Herstellung der erfindungsgemäßen Allergietestträger ist sehr einfach, genauso wie die Testdurchführung.

Mit dem erfindungsgemäßen Verfahren zur In-vitro-Diagnostik von Allergien ist davon auszugehen, dass nicht nur bereits erwartete spezifische IgE-Sensibilisierungen nachweisbar werden, sondern auch eine Vielzahl von neuen IgE-vermittelten Reaktionen beschrieben werden. Dazu gehören bislang nicht erfassbare Sensibilisierungen wie am Beispiel von Sojamilch- und Honigmelonenallergikern gezeigt, die ähnlich komplexen Schimmelpilzsensibilisierungen, aber auch andere Krankheiten wie das Intoleranz Syndrom, die Polyposis nasi und Alkoholunverträglichkeiten mit Asthma bronchiale kann man nun auf spezifisches IgE hin testen.

Beispielweise konnte bei Patienten, die nach Genuss von Sojamilch einen anaphylaktischen Schock bekamen, mit dem erfindungsgemäßen nativen Sojamilch-Allergietestträger spezifisches IgE der Klasse 1,9 gemessen werden. Mit kommerziell erhältlichen Sojabohne- und Sojaschrot-Allergenscheiben wurde dagegen die Klasse 0 gemessen (siehe Beispiel 3 bzw. Tabelle 1). Das Serum eines Patienten mit einer Anaphylaxie auf Honigmelonen, zeigte bei den Verkaufsscheiben Melone (Allergopharma) und dem Immuno-Melonen-CAP (Phadia, Freiburg) nach Vorschrift des Herstellers gemessen eine RAST Klasse 0 und somit keinen Nachweis von spezifischen IgE-Antikörpern auf Melone. Aus Tabelle 1 ist zu entnehmen, dass nur mit dem erfindungsgemäßen nativen Honigmelonen-Allergietestträger eine positive Klasse (1,9) mit dem Serum des Patienten gemessen wurde. Alle drei Patienten zeigten klinisch eine Anaphylaxie, also eine lebensbedrohliche allergische Reaktion, die durch IgE-Antikörper vermittelt abläuft. Mittels der herkömmlichen serologischen Methodik war das verantwortliche spezifische IgE nicht nachweisbar. Mit der hier beschriebenen In-vitro Diagnostik mittels der erfindungsgemäßen nativen Allergietestträger lässt sich das spezifische IgE jedoch nachweisen. Die klinische Diagnose und die Ergebnisse aus der mit Risiko für den Patienten durchgeführten Hauttestung, die eindeutig positiv waren, sind nun in Übereinstimmung mit den serologischen Befunden bzw. der In-vitro-Allergiediagnostik.

Das erfindungsgemäße Verfahren zur In-vitro-Diagnostik von Allergien dient zur Beurteilung von spezifischem IgE in Vollblut oder Seren von Allergikern und kann neben dem Humanbereich gleichermaßen auch im Veterinärbereich eingesetzt werden.

Ein weiterer Gegenstand der Erfindung ist ein Verfahren zur In-vitro-Diagnostik von Proteinen, **dadurch gekennzeichnet, dass**
a.) ein aktivierter Träger mit einem Proteine enthaltenden Stoff in Kontakt gebracht wird,
b.) die Proteine an den aktivierten Träger koppeln,
c.) nach Kopplung der Proteine an den aktivierten Träger keine Blockierung erfolgt
d.) und mit dem erhaltenen Proteintestträger direkt zur Messung oder zum Nachweis der gebundenen Proteine verwendet wird,
e.) oder der erhaltene Proteintestträger bis zur Messung oder zum Nachweis der gebundenen Proteine aufbewahrt wird.

Ein weiterer Gegenstand der Erfindung sind Kits, geeignet zur In-vitro-Diagnostik von Allergien, enthaltend wenigstens einen erfindungsgemäßen aktivierten Träger und ein oder mehrere Dinge ausgewählt aus der Gruppe bestehend aus Negativkontrollserum, Konjugat, Substrat, Mikrotiterstreifen, Teststreifen, Pinzetten, Fläschchen, Röhrchen, Etiketten und Allergietestträger mit daran gekoppelten Allergenen. Beispiele für erfindungsgemäße Kits sind nachfolgende Testkits 1 und 2.

Testkit 1 bestehend aus:
- Fläschchen mit lyophiliserten BrCN-aktivierten Papierscheiben
- Fläschchen mit Aufbewahrungspuffer (Allergenscheibenpuffer)
- Glas- oder Kunststofffläschchen mit Schraubverschluss
- Etiketten
- Kunststoffpinzette
- Fläschchen mit Negativpoolserum

Testkit 2 - wie Testkit 1 jedoch zusätzlich:
- Fläschchen mit Konjugat (aus Allergodip^{®})
- Fläschchen Substrat (aus Allergodip^{®})
- Teststreifen mit zwei bis drei BrCN-aktivierten Papierpads

### Gesamt-IgE-Messung

Obwohl die Bestimmung der Höhe des Gesamt-IgE-Spiegels in Allergikerseren in der Allergiediagnostik weniger aussagefähig ist als die Bestimmung des spezifischen IgE-Spiegels, hat die Bestimmung des Gesamt IgE einen festen Platz in der Laboratoriumsdiagnostik behalten können. Untersuchungen zeigten, dass der Gesamt-IgE-Spiegel nicht unbedingt mit dem spezifischen IgE-Spiegel korrelieren muss, das heißt ein hoher Gesamt-IgE-Spiegel geht nicht gleichzeitig auch mit Vorliegen hoher spezifischer IgE-Spiegel einher. Dennoch ist der Gesamt-IgE-Spiegel ist bei Typ I-Allergien, parasitären Erkrankungen und seltenen Systemerkrankungen von diagnostischer Bedeutung. Auch hat die Gesamt-IgE-Untersuchung einen gewissen Stellenwert bei der Untersuchung von Medikamentenallergikern.

### Spezifische IgE-Messung

Von weit größerer Aussagekraft bezüglich einer vorliegenden Allergie - bzw. des Sensibilisierungsgrades - ist die Bestimmung des spezifischen IgE-Spiegels im Serum bzw. Plasma des Patienten. Die quantitative Bestimmung des zirkulierenden spezifischen IgE im Serum bzw. Plasma erfolgt durch einen nicht kompetitiven Enzymimmunoassay. Die Festphase kann aus einem chemisch aktivierten Träger, beispielsweise einer BrCN-aktivierten Papierscheibe bestehen, an die die entsprechenden Allergene kovalent gebunden sind. Die Auswertung des Ergebnisses erfolgt über eine Standardkurve, mit den Extinktionswerten der gemessenen Referenzen. Üblich sind die Klassenunterteilungen 0 - 5 oder 0 - 6, wobei Klassen 5/6 den höchsten spezifischen IgE-Spiegeln im Serum und Klasse 0 den niedrigstem Spiegel entsprechen. Der klinische cut-off kann von Allergen zu Allergen durchaus verschieden sein. Allgemein kann aber davon ausgegangen werden, dass ab einer Klasse ≥ 2 von klinischer Seite Handlungsbedarf besteht, wie beispielsweise die Einleitung einer spezifischen Immuntherapie oder einer kausalen Behandlung des Patienten.

Bei der Interpretation der Ergebnisse ist großer allergologischer Sachverstand erforderlich. Aus diesem Grunde sollte die Interpretation entsprechend der Empfehlung der ÄDA von einem erfahrenen Allergologen durchgeführt werden. Zur Herstellung der Referenzreihe, gegen den der Abgleich des Messwertes der Proben erfolgt, wird ein hochtitriges Gesamt-IgE-Serum (z.B. Myeloma IgE), durch Verdünnung auf die gewünschten Konzentrationen (IU/ml) eingestellt, die z.B. den Klassen 1-5 entsprechen. Die meisten spezifischen IgE-Systeme sind gegen den Gesamt IgE WHO-Standard kalibriert und umschreiben den Bereich von Klasse 1 (0,35 IU/ml) bis Klasse 5 (50 IU/ml) bzw. 6 (100 IU/ml). Da es keinen internationalen Standard für spezifisches IgE gibt, sollte das Ergebnis sinnvollerweise nicht in IU/ml (international Units/ml) ausgedrückt werden, sondern in U/ml (Units/ml) oder KU_{(A)}/I (Kilo Units/I) (_{A}=allergenspezifisch).

Neben IgE-Antikörpern befinden sich noch weitere Immunglobuline im Serum. Im Serum von Nichtallergikern kommt IgE in den niedrigsten Konzentrationen (17-450 ng/ml) und IgG in den höchsten Konzentrationen (8-16 mg/ml) aller Immunglobuline vor. Die anderen aufgeführten Immunglobuline spielen in der Allergiediagnostik keine besondere Rolle.

Der Nachweis von spezifischem IgE in Vollblut oder Seren erfolgt beispielweise gemäß nachfolgendem Protokoll:
1. Pipettieren von Vollblut oder Serum in ein Teströhrchen;
2. Inkubieren mit einem erfindungsgemäßen Allergietestträger;
3. Waschen unter fließendem Wasser;
4. Kurzes Trocknen;
5. Inkubation des Allergentestträgers mit Enzym-Anti-IgE-Konjugat;
6. Waschen;
7. Kurzes Trocknen;
8. Inkubieren mit Enzym-Substrat;
9. Kurzes Trocknen mit Fließpapier;
10. Entnahme des entwickelten Teststreifens;
11. Abgleich von Positiv mit Negativknotrolle;
12. Abgleich gegen eine Farbkarte oder Abgleich und Dokumentation mittels der kombinierten Patienten-Farbkarte

Die Auswertung erfolgt vorzugsweise visuell durch Vergleich mit einer Farbkarte. Dabei werden die entstehenden Färbungen der Allergenpads auf dem jeweiligen Teststreifen mit einer vorgegebenen Farbskala verglichen. Die Farbintensität ist dabei der Indikator für den Befund der Allergiediagnose.

Zusammenfassend lässt sich sagen, dass der Einsatz der erfindungsgemäßen Allergietestträger besonders bei der Nahrungsmittelallergie-Diagnostik eine verlässlichere In-Vitro-Diagnostik als die bisher bekannten ermöglicht und bislang nicht erfassbare Sensibilisierungen erfassen kann. Die Herstellung ist sehr einfach, genauso wie die Testdurchführung und deshalb kann der Arzt die Herstellung und die Testdurchführung selbst und direkt vor Ort durchführen.

### Beispiel 1 - Vergleich der Kopplungsarten

| **Herstellung nach Ceska et al.** | **Erfindungsgemäßer nativer Allergietestträger, Herstellung nach R. Wahl** | **Bemerkung** |
|---|---|---|
| Inkubation der BrCN aktivierten Papierscheibe mit dem Nahrungsmittelextrakt (L02): Überkopfschüttler. 2-8°C 15-17 Std. Danach L02 abdekantieren | Auflegen des BrCN aktivierten Papierscheibe auf das Nahrungsmittel z.B. Melone. Inkubation 1-5 Minuten. Eintauchen der BrCN aktivierten Papierscheibe in die Flüssigkeit, z.B. Kuhmilch. 5 Sekunden genügen. | Die nativen Allergenscheiben können direkt im Test eingesetzt werden oder unter Allergenscheibenpuffer (L10) bei 2-8°C zwischen gelagert werden, bis zum Testeinsatz. |
| Waschung der Scheiben mit einem Ethanolamin-haltigen Puffer L03. Bei 2-8°C einmal 10 Min und anschließend 3 Std. Überkopfschüttler L03 jeweils durch Abdekantieren (Sieb vorhalten) entfernen. | Entfällt | |
| Waschung mit einem Essigsäure-haltigen Puffer (L06). 3 mal bei 2-8°C jeweils 10 Min. Überkopfschüttler | Entfällt | |
| Waschung 2 mal mit L10 bei 2-8°C jeweils 10 Min. Überkopfschüttler. L10 durch Abdekantieren (Sieb vorhalten) entfernen. L10 durch abtropfen lassen soweit möglich entfernen. | Entfällt | |
| Lagerung der Scheiben unter Allergenscheibenpuffer (L10) | Lagerung der Scheiben unter Allergenscheibenpuffer oder Direkteinsatz im Test. | |

Lösungen:
L02: 9,00 g NaCl, 0,50g NaN3, auf 1000 ml AP-Wasser
L03: 8,40g NaHCO3, 3,05 g Ethanolamin auf 1000 ml AP-Wasser
L06: L04 vorlegen: 6,26 g Essigsäure auf 1000 ml AP-Wasser vorlegen, L05 (13,61 g Natriumacetat) auf 1000 ml L6: L4 mit L5 auf pH 4,0 eingestellt.
L10: 9,00 g NaCl, 3,00 g Humanserumalbumin, 0,50 g NaN₃ auf 1000 ml L09: L07 vorlegen L08 bis pH 7,4 zugeben.
L07: 8,71 g K2HPO4 auf 1000 ml AP-Wasser
L08: 6,80 g KH2PO4 auf 1000 ml

Aus dem obenstehenden Vergleich ergibt sich, dass die Herstellung erfindungsgemäßer Allergietestträger in sehr kurzer Zeit und sehr einfach selbst für einen Arzt ausführbar ist. Es sind keine umfangreichen Inkubationen und Waschungen notwendig.

### Beispiel 2 - Herstellung der erfindungsgemäßen nativen Allergietestträger

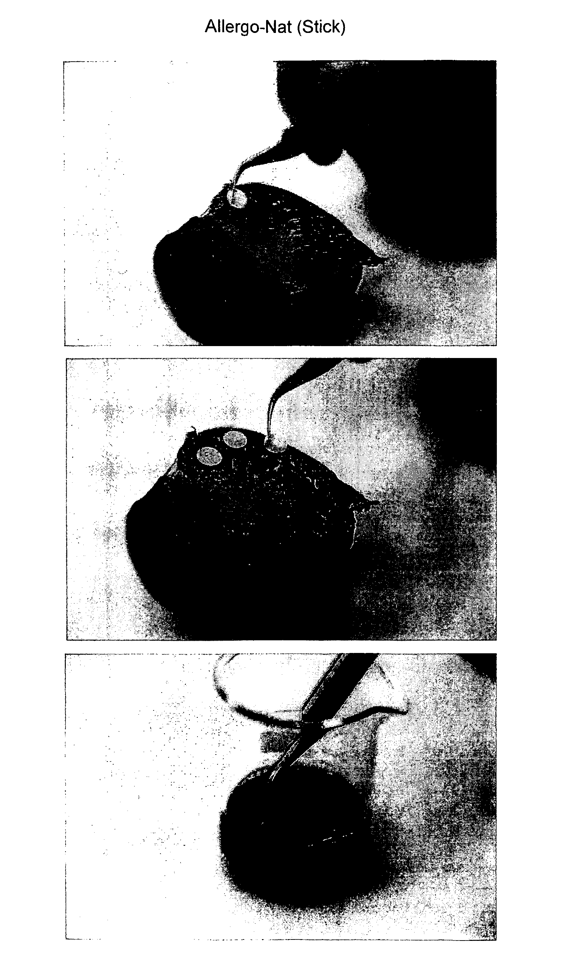

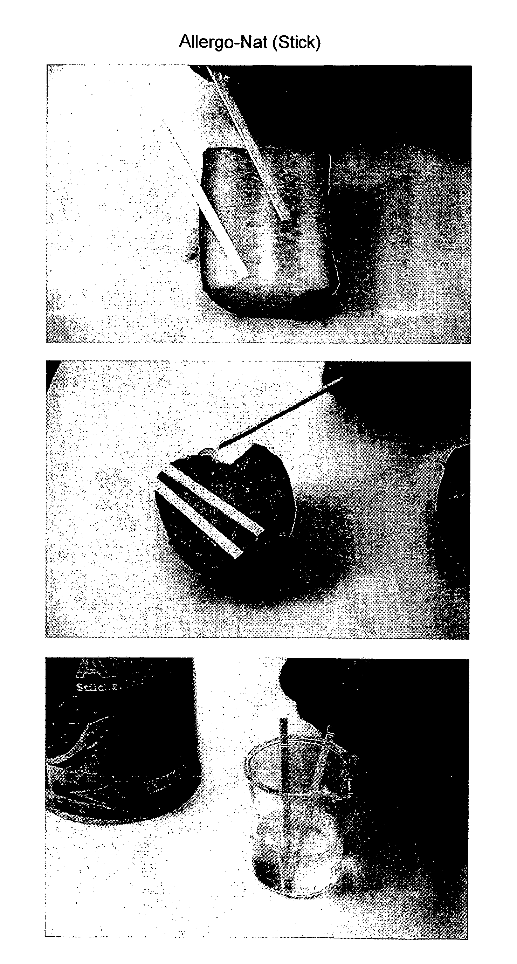

Erfindungsgemäße native Allergentestträger wurden mit Honigmelone, Wassermelone, Kiwi, Pfirsich, Apfel, Banane, Zwiebel, Rindfleisch und Apfelsaftschorle hergestellt. Die Herstellung erfolgte, indem BrCN-aktivierte Papierscheiben (Allergopharma) beispielsweise für fünf Minuten bei Raumtemperatur mit einer Pinzette auf Fruchtfleisch gelegt und etwas angedrückt wurden, sodass die Scheibe komplett mit dem Fruchtfleisch in Kontakt kam. Es war leicht zu erkennen, ob die chemisch aktivierte Papierscheibe mit dem Fruchtfleisch in Kontakt war, da die Scheibe bei vollständiger Benetzung mit dem Saft einen homogenen, visuell sehr gut erkennbaren Helligkeitsunterschied zeigte (Abbildung 1).
Nach kurzer Inkubation mit dem Allergene enthaltenden Stoff wurden die nativen Allergietestträger in einen Aufbewahrungspuffer gegeben und bis zum Einsatz im Test bei 2-8°C gelagert.

### Tomate:

Eine Tomate wurde durchgeschnitten und über den gesamten Bereich (flüssig/fest) wurden BrCN-aktivierte Papierscheiben verteilt und fünf Minuten bei Raumtemperatur inkubiert. Sie saugten sich sofort deutlich erkennbar mit Flüssigkeit voll. Danach wurden die Allergietestträger in Aufbewahrungspuffer gegeben und bis zum Einsatz im Test bei 2-8°C gelagert.

### Apfelsaftschorle, Kuhmilch, Sojamilch:

BrCN-aktivierte Papierscheiben wurden etwa 10 bis 15 Sekunden mit einer Pinzette in Apfelschorle, Kuhmilch oder Sojamilch gehalten und bis zum Einsatz im Test in Aufbewahrungspuffer bei 2-8°C gelagert.

### Kiwi, Banane, Zwiebel, Pfirsich, Birne, Orange, Kartoffel:

Die Frucht wurde durchgeschnitten und BrCN-aktivierte Papierscheiben sofort in Kontakt mit dem Fruchtfleisch gebracht und fünf Minuten bei Raumtemperatur inkubiert. Danach wurden die Allergietestträger in Aufbewahrungspuffer gegeben und bis zum Einsatz im Test bei 2-8°C gelagert.

### Rindfleisch:

BrCN-aktivierte Papierscheiben sofort in Kontakt mit dem Fruchtfleisch gebracht und fünf Minuten bei Raumtemperatur inkubiert. Danach wurden die Allergietestträger in Aufbewahrungspuffer gegeben und bis zum Einsatz im Test bei 2-8°C gelagert.

### Bier (Flensburger):

BrCN-aktivierte Papierscheiben wurden etwa 5 bis 10 Sekunden mit einer Pinzette in Bier gehalten. Es kam zu leichtem Sprudeln. Danach wurden die Allergietestträger in Aufbewahrungspuffer gegeben und bis zum Einsatz im Test bei 2-8°C gelagert.

### Rotwein (Merlot):

BrCN-aktivierte Papierscheiben wurden etwa 5 bis 10 Sekunden mit einer Pinzette in Wein gehalten. Die Scheiben färbten sich dabei etwas. In Scheibenpuffer. Danach wurden die Allergietestträger in Aufbewahrungspuffer gegeben und bis zum Einsatz im Test bei 2-8°C gelagert.

### Penicillin und Amoxicillin:

Es wurden Penicillin- bzw. Amoxicillin-Tabletten zermörsert und in physiologische Kochsalzlösung aufgenommen, mehrfach leicht geschwenkt und etwa 30 Minuten stehen gelassen. Dann wurden entweder BrCN-aktivierte Papierscheiben oder HSA-aktivierte Papierscheiben kurz in die Suspension gehalten. Danach wurden die Allergietestträger in Aufbewahrungspuffer gegeben und bis zum Einsatz im Test bei 2-8°C gelagert.

### Teststreifen:

Auf einen Papierstreifen wurden zwei oder drei BrCN-aktivierte Papierpads geklebt (zur Doppel- oder Dreifachbestimmung). Dann wurde der Teststreifen mit den Plättchen für fünf Minuten auf das Fruchtfleisch gelegt (Tomate bzw. Banane), danach mit Fliespapier leicht abgetupft und in ein Röhrchen gegeben und bis zum Einsatz im Test bei 2-8°C in gelagert.

### Tierspeichel:

Man ließ eine Katze Teststreifen, auf denen sich zwei BrCN-aktivierte Papierpads befinden, ablecken. Danach wurden die Allergietestträger in ein Röhrchen gegeben und bis zum Einsatz im Test bei 2-8°C gelagert.

### Tierhaare und -Epithelien:

Leicht angefeuchtete Teststreifen, auf denen sich zwei BrCN-aktivierte Papierpads befinden, wurde einer Katze über das Fell gezogen oder gestrichen. Dann gab man die Teststreifen in ein Teströhrchen. Zur Überprüfung der Reproduzierbarkeit wurden drei Teststreifen eingesetzt. Danach wurden die Allergietestträger in ein Röhrchen gegeben und bis zum Einsatz im Test bei 2-8°C gelagert.

### Beispiel 3 - Vergleich des gemessenen spezifischen IgE ermittelt mit erfindungsgemäßen nativen Allergietestträgern und kommerziell erhältlichen Allergenscheiben

### Patientengut:

Seren von entsprechend sensibilisierten Patienten wurden mit erfindungsgemäßen nativen Allergietestträgern und den entsprechenden kommerziell erhältlichen Allergenscheiben gemessen. Ferner wurde ein Serum eines Honigmelonenallergikers gemessen, der nach dem Genuss von Honigmelone schwere Schwellungen bekam und im Prick zu Prick-Test eine stark ausgeprägte Hautreaktion aufwies, sowie zwei Seren von Patienten, die nach dem Genuss von Sojamilch einen anaphylaktischen Schock bekamen. Als Negativkontrolle wurde ein Nichtallergikerpoolserum (N=5) mit den entsprechenden Allergenträgern parallel gemessen. Die Messung des spezifischen IgE bzw. der Allergenscheibentest (Allergopharma), wurde nach Vorschrift des Herstellers durchgeführt. Das Ergebnis wurde in Klassen ausgedrückt. Die Messung erfolgte in Doppelbestimmung (VK% < 5%)

**Tabelle 1**

| Vergleich - Klassen gemessen mit kommerziell erhältlichen Allergenscheiben und erfindungsgemäßen nativen Allergietestträgern. NK: Negativkontrollserum | | | |
|---|---|---|---|
| **Seren** | **Allergen** | **kommerziell erhältliche Allergenscheibe** | **erfindungsgemäßer nativer Allergietestträger** |
| | | | |
| 5750 | Banane | 3,0 | 3,0 |
| 5620 | Banane | 2,5 | 2,3 |
| 5313 | Banane | 2,5 | 2,4 |
| 5711 | Banane | 2,2 | 2,1 |
| NK | Banane | 0 | 0 |
| | | | |
| 5645 | Zwiebel | 2,5 | 2,2 |
| 5664 | Zwiebel | 3,0 | 2,2 |
| 5713 | Zwiebel | 3,0 | 2,2 |
| 5313 | Zwiebel | 3,3 | 2,3 |
| 5645 | Zwiebel | 2,5 | 2,2 |
| NK | Zwiebel | 0 | 0 |
| | | | |
| 5749 | Kiwi | 2,1 | 3,5 |
| 5752 | Kiwi | 2,2 | 1,8 |
| 5629 | Kiwi | 2,1 | 2,1 |
| 5750 | Kiwi | 3,0 | 3,2 |
| 5564 | Kiwi | 2,2 | 2,1 |
| NK | Kiwi | 0 | 0 |
| | | | |
| 5718 | Honigmelone Ink. 1Min | 2,5 (Melone) | 2,3 |
| J.L | Honigmelone Ink. 1 Min. | 0 (Melone)/CAP: 0 | 1,9 |
| NK | Honigmelone | 0 (Melone) | 0 |
| | | | |
| 5718 | Wassermelone | 2,1 | 2,1 |
| M11/3 | Wassermelone | 2,5 | 2,2 |
| NK | Wassermelone | 0 | 0 |
| | | | |
| 5752 | Pfirsich | 4,2 | 2,0 |
| NK | Pfirsich | 0 | 0 |
| | | | |
| 5313 | Orange | 3,0 | 2,1 |
| 5493 | Orange | 2,1 | 2,1 |
| 5735 | Orange | 2,1 | 2,5 |
| 5749 | Orange | 2,7 | 2,3 |
| 5750 | Orange | 3,8 | 3,1 |
| NK | Orange | 0 | 0 |
| | | | |
| | | | |
| 5313 | Apfel | 2,3 Mischung Granny Smith / Golden delicious | 2,3 Granny Smith |
| 5572 | Apfel | 2,1 | 2,1 |
| 5712 | Apfel | 3,4 | 3,1 |
| 5713 | Apfel | 2,2 | 2,2 |
| 5749 | Apfel | 3,1 | 3,8 |
| 5750 | Apfel | 3,1 | 3,1 |
| 5752 | Apfel | 3,3 | 2,1 |
| 5748 | Apfel | 3,3 | 2,1 |
| NK | Apfel | 0 | 0 |
| | | | |
| 5748 | Tomate | 3,0 | 2,3 |
| NK | Tomate | 0 | 0 |
| | | | |
| M11/25 | Kartoffel | 2,4 | 2,3 |
| NK | Kartoffel | 0 | 0 |
| | | | |
| M1172 5 | Kuhmilch | 2,6 | 1,7 |
| NK | Kuhmilch | 0 | 0 |
| | | | |
| BB2816 | Sojabohne, Sojaschrot, Sojamilch | 0 (Sojabohne) 0 (Sojaschrot) | 1,9 Sojamilch (kommerziell nicht erhältlich) |
| BB2813 | Sojabohne, | 0 (Sojabohne) | 1,9 Sojamilch |
| | Sojaschrot, | 0 (Sojaschrot) | (kommerziell nicht erhältlich) |
| | Sojamilch | | |
| BB 2825 | Sojabohne, | 0 (Sojabohne) | 1,4 Sojamilch |
| | Sojaschrot, | 0 (Sojaschrot) | (kommerziell nicht erhältlich) |
| | Sojamilch | | |
| NK1 | Sojamilch | | 0 |
| NK2 | Sojabohne, | | 0 |
| NK2 | Sojaschrot | | 0 |

Aus der Tabelle 1 ist zu entnehmen, dass mit erfindungsgemäßen nativen Allergietestträgern vergleichbare Ergebnisse zu mit kommerziell erhältlichen Allergenscheiben erhaltenen Ergebnissen ermittelt wurden.

### Messung eines klinisch eindeutig positiven Honigmelonenallergikers

Bei einem Patienten, bei dem nach dem Genuss einer Honigmelone schwere Mundschleimhautschwellungen auftraten, wurde ein Scratch-Test durchgeführt mit positivem Ergebnis. Die In-vitro-Diagnostik ergab sowohl bei der Messung mit der Allergenscheibe Melone als auch mit dem CAP Melone ein eindeutig negatives Resultat, obwohl die klinischen Beschwerden und die Hauttestung eindeutig positiv waren. Das Serum des Patienten wurde mit den erfindungsgemäßen nativen Honigmeionen-Allergietestträgern, den kommerziell erhältlichen Allergenscheiben Melone (Allergopharma) und dem Immuno-CAP (Phadia, Freiburg) nach Vorschrift des Herstellers mit dem Melonen-CAP gemessen. Aus Tabelle 1 ist zu entnehmen, dass nur mit dem erfindungsgemäßen nativen Honigmelonen-Allergietestträger eine positive Klasse (1,9) mit dem Serum des Patienten gemessen wurde. Sowohl mit der kommerziell erhältlichen Allergenscheibe Melone als auch mit dem CAP (Melone) wurde die Klasse 0 gemessen.

### Messung dreier Seren von Patienten, die nach dem Genuss von Sojamilch einen anaphylaktischen Schock bekamen

Mit dem erfindungsgemäßen nativen Sojamilch-Allergietestträger wurden die Klassen 1,9 bzw. 1,4 gemessen. Mit den kommerziell erhältlichen Sojabohne- und Sojaschrot-Allergenscheiben wurde dagegen die Klasse 0 gemessen (siehe Tabelle 1)

### Beispiel 4 - Überprüfung der Inkubationszeit zur Herstellung der erfindungsgemäßen nativen Allergietestträger

Zur Überprüfung der optimalen Kopplungsbedingungen wurde mit Honigmelone eine Inkubation von 1, 2, 3, 5 und 10 Minuten vorgenommen, das heißt, BrCN-aktivierte Papierscheiben wurden für 1, 2, 3, 5 und 10 Minuten auf das Fruchtfleisch gelegt. Ein entsprechendes Serum eines auf Honigmelonen sensibilisierten Patienten wurde mit diesen nativen Allergenscheiben im EAST gemessen.

**Tabelle 2**

| Herstellung der erfindungsgemäßen nativen Melonen-Allergenscheiben über unterschiedliche Inkubationszeiten, d.h. unterschiedlich langer Kontakt der BrCN aktivierten Papierscheibe mit dem Fruchtfleisch, Klassenmessung. | | | |
|---|---|---|---|
| **Serum** | **Allergenscheibe** | **Inkubationszeiten (Min)** | **Klassen Messung** |
| 5718 | Honigmelone | 1 | 2,3 |
| | Honigmelone | 2 | 2,7 |
| | Honigmelone | 3 | 2,5 |
| | Honigmelone | 5 | 2,9 |
| | Honiqmelone | 10 | 2,8 |

Die Messung Honigmelone zeigt, dass schon eine Inkubation der BrCN-aktivierten Papierscheibe mit dem Honigmelonenfruchtfleisch von einer Minute genügt, um die Scheibe für die EAST-Messung zu aktivieren.

### Beispiel 5 - Untersuchung zur reproduzierbaren Messung mit den erfindungsgemäßen nativen Allergietestträgern

Die Reproduzierbarkeit der Messung wurde mit den erfindungsgemäßen nativen Honigmelonen- und Kiwi-Zwiebel und Apfel-Allergietesträgern mit entsprechenden Seren von entsprechend sensibilisierten Patienten überprüft. Es wurden jeweils drei unabhängige Messungen zeitversetzt in Doppelbestimmung durchgeführt. Die Klassen wurden miteinander verglichen.

**Tabelle 3**

| Reproduzierbare Messung (Klassen) durchgeführt mit erfindungsgemäßen nativen Honigmelonen- und Kiwi-Allergietesträgern | | | | |
|---|---|---|---|---|
| **Serum** | **Allergen** | **Messung 1** | **Messung 2** | **Messung 3** |
| 5718 | Honigmelone | 2,1 | 2,5 | 2,4 |
| M11/23 | Honigmelone | 2,5 | 2,1 | 2,3 |
| | | | | |
| 5629 | Kiwi | 2,2 | 1,6 | 2,1 |
| 5749 | Kiwi | 3,1 | 3,0 | 3,5 |
| | | | | |
| 5752 | Pfirsich | 2,0 | 2,7 | 2,2 |
| M11/9 | Pfirsich | 1,8 | 2,1 | 2,0 |

Aus Tabelle 3 ist die sehr gute reproduzierbare Messung zu entnehmen.

Mit demselben Ergebnis wurden Versuche mit Teststreifen durchgeführt die drei BrCN-aktivierte Papierpads trugen. Diese Teststreifen wurde auf das Fruchtfleisch von Tomaten gelegt, fünf Minuten inkubiert und in Aufbewahrungspuffer bei 2-8°C über vier Tage gelagert und dann im Test eingesetzt.

**Tabelle 3.1**

| **Teststreifen** Tomate Serum 5748 | **Pad 1** **Klasse** | **Pad 2** **Klasse** | **Pad 3** **Klasse** |
|---|---|---|---|
| 1 | 2 | 2 | 2 |
| 2 | 2 | 2 | 2 |

Auch hier ergab sich eine hohe Reproduzierbarkeit.

### Beispiel 6 - Untersuchung der reproduzierbaren Herstellung der erfindungsgemäßen nativen Allergietestträger

Die Reproduzierbarkeit der Herstellung der erfindungsgemäßen nativen Allergietestträger wurde in jeweils zwei Ansätzen mit Zwiebel, Kiwi, Apfel (Granny Smith) und Honigmelone überprüft. Entsprechende Seren wurden mit diesen Allergenscheiben gemessen und die ermittelten Klassen wurden verglichen.

**Tabelle 4**

| Reproduzierbare Herstellung der erfindungsgemäßen nativen Allergietestträger. Inkubation 5 Min., Klassenmessung | | | |
|---|---|---|---|
| **Serum** | **Allergenscheibe** | **Herstellung 1** | **Herstellung 2** |
| 5645 | Zwiebel | 2,2 | 2,5 |
| 5713 | Zwiebel | 2,1 | 2,5 |
| | | | |
| 5749 | Kiwi | 3,3 | 3,0 |
| | | | |
| 5313 | Apfel (Granny Smith) | 2,1 | 2,3 |
| | | | |
| 5718 | Honigmelone | 2,3 | 2,5 |
| M11/23 | Honigmelone | 2,5 | 2,4 |

Am Beispiel von Zwiebel, Kiwi, Apfel und Honigmelone konnte, aufgrund der Klassenmessung, eine gute Reproduzierbarkeit der Herstellung der nativen Allergenscheiben gezeigt werden.

### Beispiel 7 - Untersuchung des Einflusses von Ethanolamin bei der Herstellung Allergietestträgern

Ferner wurden drei verschiedene Allergenscheiben so hergestellt, dass in einem Waschschritt kein Ethanolamin zur Blockierung zugesetzt wurde. Seren von entsprechend sensibilisierten Patienten wurden mit diesen Allergenscheiben im EAST gemessen.

**Tabelle 5**

| Vergleich Klassen gemessen mit Allergenscheiben, die mit Ethanolamin während der Herstellung blockiert und die nicht mit Ethanolamin blockiert wurden. | | | |
|---|---|---|---|
| **Seren** | **Allergenscheibe** | **Klassen mit Ethanolamin-Blockierung der Allergenscheibe** | **Klassen ohne Ethanolamin-Blockierung der Allergenscheibe** |
| 5712 | Apfel | 3,1 | 3,1 |
| 5713 | Apfel | 2,5 | 2,1 |
| 5749 | Apfel | 3,0 | 3,1 |
| 5750 | Apfel | 3,2 | 3,2 |
| 5752 | Apfel | 3,0 | 2,3 |
| 5718 | Apfel | 3,2 | 3,1 |
| 5748 | Apfel | 3,0 | 2,7 |
| NK | Apfel | 0 | 0 |
| 5495 | Bohne weiß | 3,0 | 2,7 |
| 5498 | Bohne weiß | 2,2 | 2,2 |
| 5629 | Bohne weiß | 2,5 | 2,7 |
| 5645 | Bohne weiß | 2,7 | 2,6 |
| 5679 | Bohne weiß | 3,0 | 3,0 |
| 5711 | Bohne weiß | 3,1 | 3,1 |
| 5717 | Bohne weiß | 3,0 | 2,8 |
| 5773 | Bohne weiß | 3,0 | 3,0 |
| NK | Bohne weiß | 0 | 0 |
| 5645 | Walnuß | 2,3 | 2,2 |
| 5735 | Walnuß | 2,2 | 2,4 |
| 5750 | Walnuß | 3,3 | 3,2 |
| 5751 | Walnuß | 3,0 | 3,0 |
| NK | Walnuß | 0 | 0 |

Aus Tabelle 5 ist zu ersehen, dass eine Blockierung mit Ethanolamin bei der Herstellung der Allergenscheiben nicht notwendig ist. Mit den Allergenscheiben, die ohne Blockierung mit Ethanolamin hergestellt wurden, wurden zu den mit Ethanolamin blockierten Scheiben vergleichbare Klassen gemessen.

### Beispiel 8 - Test auf unspezifische Reaktion

Ein Myeloma IgE-Serum, das auf 0, 500 und 1000 IU/ml eingestellt wurde, sollte mit verschiedenen erfindungsgemäßen nativen Allergentestträgern gemessen werden.

**Tabelle 6**

| Messung Myeloma IgE-Serum (0, 500,1000 IU/ml) mit verschiedenen nativen Allergenscheiben | | |
|---|---|---|
| **IU/ml** | **Native Allergenscheibe** | **Klasse** |
| 0 | Banane | 0 |
| 500 | Banane | 0 |
| 1000 | Banane | 0 |
| | | |
| 0 | Oranqe | 0 |
| 500 | Orange | 0 |
| 1000 | Orange | 0 |
| | | |
| 0 | Pfirsich | 0 |
| 500 | Pfirsich | 0 |
| 1000 | Pfirsich | 0 |
| | | |
| 0 | Kartoffel | 0 |
| 500 | Kartoffel | 0 |
| 1000 | Kartoffel | 0 |
| | | |
| 0 | Kuhmilch | 0 |
| 500 | Kuhmilch | 0 |
| 1000 | Kuhmilch | 0 |
| | | |
| 0 | Tomate | 0 |
| 500 | Tomate | 0 |
| 1000 | Tomate | 0 |
| | | |
| 0 | Kiwi | 0 |
| 500 | Kiwi | 0 |
| 1000 | Kiwi | 0 |
| | | |
| 0 | Birne | 0 |
| 500 | Birne | 0 |
| 1000 | Birne | 0 |
| | | |
| 0 | Zwiebel | 0 |
| 500 | Zwiebel | 0 |
| 1000 | Zwiebel | 0 |

Aus Tabelle 6 geht hervor, dass bei Messung mit unterschiedlichen erfindungsgemäßen nativen Allergentestträgern immer eine Klasse 0 und damit keine unspezifische Reaktion gemessen wurde.

### Beispiel 9 - Stabilität der erfindungsgemäßen nativen Allergietestträger

Die erfindungsgemäßen nativen Allergentestträger Zwiebel und Kiwi wurden unter Allergenscheibenpuffer bei 2-8°C bis zu elf Wochen gelagert.

**Tabelle 7**

| Stabilitätsdaten, Lagerung unter Allergenscheibenpuffer bei 2-8°C | | |
|---|---|---|
| **Native Allergenscheibe** | **Zeit** | **Klasse** |
| Kiwi | Tag 0 | 2,0 |
| Kiwi | 11 Wochen | 2,1 |
| | | |
| Zwiebel | Tag 0 | 2,0 |
| Zwiebel | 11 Wochen | 2,0 |
| | | |
| Gurke grün | Tag 0 | 2,2 |
| Gurke grün | 2 Wochen | 2,4 |

Aus den Daten zur Stabilität geht hervor, dass die erfindungsgemäßen nativen Allergentestträger, gelagert in Allergenscheibenpuffer bei 2-8°C, wenigstens elf Wochen stabil sind, was sich an den vergleichbaren Klassen zu Tag 0 zeigte (siehe Tabelle 7). Demnach ist zu erwarten, dass erfindungsgemäße native Allergentestträger auch deutlich länger aufbewahrt werden können.

### Beispiel 10 - Nachweis von spezifisichem IgE auf Tierurin

**Tabelle 8 - Rattenurin**

| **Seren** | **Dip-Klasse (Herstellung nativ)** | **Native Allergenscheibe, Klasse** |
|---|---|---|
| M07/19 (Negativkontrolle) | 0 | 0 |
| M11/23 | 3 | 3,0 |
| M11/25 | 2 | 2,1 |

**Tabelle 9 - Leguanurin**

| **Serum** | **DIP-Klasse (Herstellung nativ)** |
|---|---|
| M07/19 (Negativkontrolle) | 0 |
| P: Messung 1 | 3 |
| P. Messsung 2 | 3 |

Mittels der nativen Testung konnte erstmals nachgewiesen werden, dass Leguanurin ein Allergen darstellt.

### Beispiel 11 - Einsatz des individuellen Allergenträgers zur Identifizierung von Schimmelpilzen auf Nahrungsmitteln, z.B. Ananas

**Tabelle 10 - Schimmelpilze auf Nahrungsmitteln**

| **Seren** | **Dip-Klasse (native Herstellung)** |
|---|---|
| M07/19 (Negativkontrolle) | 0 |
| 5729 positiv auf Alternaria tenuis | 0 |
| 5737 positiv auf Botrytis cinera | 3 |
| 5729 positiv auf Phoma betae | 0 |
| 5625 positiv auf Pullularia pullulans | 2 |

Mittels der neuen nativen Technik konnte der Schimmelpilz auf Ananas durch Kombination mit entsprechenden, positiven Seren nachgewiesen werden (Botrytis cinera, Pullularia pullulans).

### Beispiel 12 - Messung von spezifischem IgE auf gekochte Nahrungsmittel wie Hühnerei und Kartoffel

**Tabelle 11 - gekochten Nahrungsmittel**

| **Seren** | **Nativer Stick** | **Klasse** |
|---|---|---|
| M2005/05 Nichtallergiker | Hühnereiweiß gekocht (fest) | 0 |
| BB 2827 | | 2/3 |
| BB 2827 | | 2/3 |
| BB 2828 | | 1 |
| | | |
| M 2005/05 | Hühnerei gekocht ( Glibber über dem Eigelb) | 0 |
| BB 2827 | | 1 |
| BB 2827 | | 1 |
| BB 2828 | | 0 |
| | | |
| M2005/05 | Gekochte Kartoffel | 0 |
| 5750 | | 2 |

Durch die neue Technologie konnte erstmals ein spezifischer IgE-Wert auf gekochte Nahrungsmittel nachgewiesen werden.

### Beispiel 13 - Validierung

Von einer grünen Gurke wurden zehn Scheiben abgeschnitten. Auf jede Gurkenscheibe wurden zehn BrCN-Scheiben und zehn Sticks gelegt. Entsprechende Seren wurden dann damit im Allergodip ® und im RV-5 (Scheibensystem) gemessen.

**Tabelle 12 - Validierung**

| **Serum** | **Scheibe/Stick** | **1** | **2** | **3** | **4** | **5** | **6** | **7** | **8** | **9** | **10** |
|---|---|---|---|---|---|---|---|---|---|---|---|
| M07/19 | Scheibe | 0 | 0 | 0 | 0 | 0 | 0 | 0 | 0 | 0 | 0 |
| M11/25 | Scheibe | 2,1 | 2,4 | 2,2 | 2,2 | 2,4 | 2,3 | 2,2 | 2,2 | 2,3 | 2,0 |
| M11/23 | Scheibe | 2,1 | 2,3 | 2,3 | 2,3 | 2,4 | 2,3 | 2,3 | 2,2 | 2,2 | 2,3 |
| 5718 | Scheibe | 2,4 | 2,7 | 2,8 | 2,5 | 2,4 | 2,5 | 2,8 | 2,6 | 2,7 | 2,5 |
| | | | | | | | | | | | |
| M07/19 | Stick | 0 | 0 | 0 | 0 | 0 | 0 | 0 | 0 | 0 | 0 |
| M11/25 | Stick | 3 | 2 | 2 | 2 | 1/2 | 2 | 1/2 | 2 | 2 | 2 |
| M11/23 | Stick | 3 | 2 | 1/2 | 2 | 2 | 2 | 2 | 2 | 2 | 2 |
| 5718 | Stick | 3 | 3 | 3 | 3 | 3 | 3 | 3 | 3 | 3 | 3 |

Die Validierungsdaten konnten als gut bis sehr gut eingestuft werden.

### Beispiel 14 - Vergleichsdaten native Allergenscheibe, nativer Stick

| **Seren** | **Allergen** | **Native Stick Klassen** | **Native Scheiben Klassen** |
|---|---|---|---|
| 5748 | Tomate | 2 | 2,3 |
| M11/25 | | 2 | 2,0 |
| M11/23 | | 2 | 1,7 |
| Negativkontrolle (NK) | | 0 | 0 |
| | | | |
| 5645 | Zwiebel | 2/3 | 2,0 |
| 5664 | | 2/3 | 2,2 |
| 5713 | | 2 | 1,2 |
| M11/25 | | 2 | 1,4 |
| M11/23 | | 2 | 1,5 |
| NK | | 0 | 0 |
| | | | |
| M11/25 | Kiwi | 2 | 2,1 |
| M11/23 | | 2/3 | 2,0 |
| NK | | 0 | 0 |
| | | | |
| 5711 | Banane | 2 | 2,1 |
| M11/25 | | 2 | 2,1 |
| M11/23 | | 2 | 2,1 |
| NK | | 0 | 0 |
| | | | |
| M11/25 | Kartoffel | 2 | 2,4 |
| M11/23 | | 3 | 2,4 |
| 5629 | | 3 | 2,6 |
| NK | | 0 | 0 |
| | | | |
| 5718 | Honigmelone | 2 | 2,2 |
| M11/25 | | 2 | 2,2 |
| M11/23 | | 2 | 2,1 |
| NK | | 0 | 0 |
| | | | |
| M11/25 | Gurke | 3 | 2,1 |
| M11/23 | | 3 | 2,1 |
| 5718 | | 3 | 2,4 |
| NK | | 0 | 0 |

Unter der Berücksichtigung der Tatsache, dass die Auswertung des Sticks visuell erfolgte, während die Daten für die Scheiben mithilfe eines Computer gestützten Systems ermittelt wurden, kann der Vergleich mit gut bewertet werden. M11/25 und M 11/23: Poolseren.

### Literatur

1. Ceska, M., U. Lundkvist: A new and simple radioimmunoassay method for the determination of IgE. Immunochem 9, 1021-1030 (1972)
2. Debelic, M., R. Wahl: In-vitro-Test: Immunglobuline E und G In Manuale allergologicum IV, 9, 1-29 (1996)
3. Große, J.W.: Neue Wege in der Allergiediagnostik: Flüssig inkubieren mit Festphase-Trenntechnik. In vitro Diagn Spec. 102, 23-27 (1990)
4. Kleine-Tebbe, J.; B. Balmer-Weber, K. Beyer et al. : In-vitro-Diagnostik und molekulare Grundlagen von IgE-vermittelten Nahrungsmittelallergien. Allergo J 32(5), 177-194 (2009)
5. Kleine-Tebbe, J., T. Fuchs, U. Lepp et al: In-vitro-Diagnostik von Nahrungsmittel-Allergien. Allergo J. 10, 333-339 (2001)
6. Laborpraxis Newsletter 2009, Spörkel O., Verfahren zum Nachweis Alleergie auslösender Stoffe gesucht (www.laborpraxis.vogel.de)
7. Oertmann, C.; Bergmann, K.-C..: Die Zunahme des pollenassoziierten oralen Allergie-Syndroms - Marker für einen Wandel innerhalb der Pollinose. Allergologie 1997; 20: 611-6
8. Pastor, C., J. Cuesta-Herrenz, B. Cases, M. Perez-Gordo, E. Figueredo, M. de las Heras, F. Vivanco: Identification of major allergens in watermelon. Int Arch Allergy Immunol. 149, 291-298 (2009)
9. Renz, H., W-M. Becker, A. Bufe, J. Kleine-Tebbe, M. Raulf-Heimsoth, J. Saloga, Th. Werfel, M. Worm: In-vitro-Allergiediagnostik. Leitlinie der DGAI in Abstimmung mit der DDG. IGGG 1 (Band 4) 72-85, (2006)
10. Röseler, S.: Allergenkunde. Praktische Allergologie. Hrsg. W. Heppt, C. Bachert. Georg Thieme Verlag Stuttgart/New York 1998.
11. Rudeschko, O., B. Fahlbusch, M. Henzgen, G. Schlenvoigt, D. Hermann, S. Vieths, L. Jäger: Investigation of the stability of apple allergen extracts. Allergy, 50, 572-580 (1995)
12. Rudeschko, O., B. Fahlbusch, M. Henzgen, G. Schlenvoigt, D. Hermann, S. Vieths, L. Jäger: Optimization of apple preparation for in vivo and in vitro diagnosis. Allergy, 50, 262-268 (1995)
13. Wahl, R. in Allergie ganz einfach 7. Auflage (s. 224), Dustri-Verlag, Deissenhofen, 2005
14. Wahl, R: Identifizierung von Allergenen in biologischem Material. In. Mönchengladbacher Allergie-Seminar, Band 6: 46-65 (1994)
15. Wide, L,H. Bennich, S.G. Johannson: Diagnosis of allergy by an in vitro test for allergen antibody. Lancet 2, 1105-1107 (1967)
16. Yman, L.: Die neue Generation der Allergie-Testung: Pharmacia CAP System. In vitro Diagn Spec 1: 18-22 (1990)
17. Wahl, R., R. Krause: Methoden der In-vitro-Allergiediagnostik und deren Stellenwert unter Berücksichtigung ihrer technischen Aspekte. Allergologie, 33/3, 121-133 (2010)

## Patentansprüche

1. Verfahren zur Herstellung eines Allergietestträgers für die In-vitro-Diagnostik**, dadurch gekennzeichnet, dass**
a.) ein aktivierter Träger mit einem Allergene enthaltenden Stoff in Kontakt gebracht wird,
b.) die Allergene an den aktivierten Träger koppeln,
c.) nach Kopplung der Allergene an den aktivierten Träger keine Blockierung erfolgt
d.) und der erhaltene Allergietestträger direkt zur Messung der Bindung von spezifischen IgE-Antikörpern aus dem Serum oder Vollblut eines Patienten verwendet wird
e.) oder der erhaltene Allergietestträger bis zur Messung der Bindung von spezifischen IgE-Antikörpern aus dem Serum oder Vollblut eines Patienten aufbewahrt wird.

2. Verfahren nach Anspruch 1, **dadurch gekennzeichnet, dass** es sich bei dem Allergene enthaltenden Stoff um Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten, oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen oder um Extrakte aus Nahrungsmitteln, Körperpflegemitteln, Teilen von Pflanzen und Pilzen, Tierhaaren, Federn, Körperflüssigkeiten, Epithelien und Ausscheidungen von Tieren, Schaben, Insektengiften, Hygienegebrauchsartikeln, Stoffen, die Haptene enthalten, oder Stoffen, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

3. Verfahren nach Anspruch 1 oder 2, **dadurch gekennzeichnet, dass** es sich bei dem Allergene enthaltenden Stoff um Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten, oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

4. Verfahren nach einem oder mehreren der Ansprüche 1 bis 3, **dadurch gekennzeichnet, dass** es sich bei dem Allergietestträger um einen nativen Allergietestträger handelt.

5. Allergietestträger für die In-vitro-Diagnostik, dadurch erhältlich, dass
a.) ein aktivierter Träger mit einem Allergene enthaltenden Stoff in Kontakt gebracht wird,
b.) die Allergene an den aktivierten Träger koppeln,
c.) nach Kopplung der Allergene an den aktivierten Träger keine Blockierung erfolgt
d.) und der erhaltene Allergietestträger direkt zur Messung der Bindung von spezifischen IgE-Antikörpern aus dem Serum oder Vollblut eines Patienten verwendet wird
e.) oder der erhaltene Allergietestträger bis zur Messung der Bindung von spezifischen IgE-Antikörpern aus dem Serum oder Vollblut eines Patienten aufbewahrt wird.

6. Verwendung eines Allergietestträgers gemäß Anspruch 5 zur In-vitro-Diagnostik von Allergien.

7. Verwendung gemäß Anspruch 6, **dadurch gekennzeichnet, dass** es sich bei den Allergien um Allergien auf Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten, oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

8. Verwendung nach Anspruch 6 oder 7, **dadurch gekennzeichnet, dass** es sich bei dem Allergietestträger für die In-vitro-Diagnostik um einen nativen Allergietestträger handelt.

9. Verfahren zur In-vitro-Diagnostik von Allergien, **dadurch gekennzeichnet, dass**
a.) ein aktivierter Träger mit einem Allergene enthaltenden Stoff in Kontakt gebracht wird,
b.) die Allergene an den aktivierten Träger koppeln,
c.) nach Kopplung der Allergene an den aktivierten Träger keine Blockierung erfolgt
d.) und mit dem erhaltenen Allergietestträger direkt eine Messung der Bindung von spezifischen IgE-Antikörpern aus dem Serum oder Vollblut eines Patienten erfolgt
e.) oder der erhaltene Allergietestträger aufbewahrt wird und anschließend eine Messung der Bindung von spezifischen IgE-Antikörpern aus dem Serum oder Vollblut eines Patienten erfolgt.

10. Verfahren nach Anspruch 9, **dadurch gekennzeichnet, dass** es sich bei den Allergien um Allergien auf Nahrungsmittel, Körperpflegemittel, Teile von Pflanzen und Pilzen, Tierhaare, Federn, Körperflüssigkeiten, Epithelien und Ausscheidungen von Tieren, Schaben, Insektengifte, Hygienegebrauchsartikel, Stoffe, die Haptene enthalten, oder Stoffe, die allergene Proteine oder Peptide enthalten, wie sie in Pflanzen, Tieren, Pilzen, Bakterien, Vieren und in Gegenständen in der Umwelt und Umgebung eines Allergikers vorkommen, handelt.

11. Verfahren nach Anspruch 9 oder 10, **dadurch gekennzeichnet, dass** es sich bei dem Allergietestträger für die In-vitro-Diagnostik um einen nativen Allergietestträger handelt.

12. Kit, geeignet zur In-vitro-Diagnostik von Allergien, enthaltend wenigstens einen aktivierten Träger und ein oder mehrere Dinge ausgewählt aus der Gruppe bestehend aus Negativkontrollserum, Konjugat, Substrat, Mikrotiterstreifen, Pinzetten, Fläschchen, Röhrchen, Etiketten und Allergietestträger mit daran gekoppelten Allergenen.

13. Verfahren zur Herstellung eines Proteintestträgers für die In-vitro-Diagnostik, **dadurch gekennzeichnet, dass**
a.) ein aktivierter Träger mit einem Proteine enthaltenden Stoff in Kontakt gebracht wird,
b.) die Proteine an den aktivierten Träger koppeln,
c.) nach Kopplung der Proteine an den aktivierten Träger keine Blockierung erfolgt
d.) und der erhaltene Proteintestträger direkt zur Messung oder zum Nachweis der gebundenen Proteine verwendet wird,
e.) oder der erhaltene Proteintestträger bis zur Messung oder zum Nachweis der gebundenen Proteine aufbewahrt wird.

14. Proteintestträger für die In-vitro-Diagnostik, dadurch erhältlich, dass
a.) ein aktivierter Träger mit einem Proteine enthaltenden Stoff in Kontakt gebracht wird,
b.) die Proteine an den aktivierten Träger koppeln,
c.) nach Kopplung der Proteine an den aktivierten Träger keine Blockierung erfolgt
d.) und der erhaltene Proteintestträger direkt eine Messung oder ein Nachweis der gebundenen Proteine erfolgt,
e.) oder der erhaltene Proteintestträger bis zur Messung oder zum Nachweis der gebundenen Proteine aufbewahrt wird.

15. Verfahren zur In-vitro-Diagnostik von Proteinen, **dadurch gekennzeichnet, dass**
a.) ein aktivierter Träger mit einem Proteine enthaltenden Stoff in Kontakt gebracht wird,
b.) die Proteine an den aktivierten Träger koppeln,
c.) nach Kopplung der Proteine an den aktivierten Träger keine Blockierung erfolgt
d.) und mit dem erhaltenen Proteintestträger direkt zur Messung oder zum Nachweis der gebundenen Proteine verwendet wird,
e.) oder der erhaltene Proteintestträger bis zur Messung oder zum Nachweis der gebundenen Proteine aufbewahrt wird.
